(19) Europäisches Patentamt
European Patent Office
Office européen des brevets

(11) **EP 2 070 529 B1**

(12) **EUROPEAN PATENT SPECIFICATION**

(45) Date of publication and mention
of the grant of the patent:
**12.10.2011 Bulletin 2011/41**

(51) Int Cl.:
*A61K 31/205* (2006.01)    *A61P 9/00* (2006.01)
*A61P 9/10* (2006.01)

(21) Application number: **08170584.0**

(22) Date of filing: **03.12.2008**

(54) **Medical use of 3-(2,2,2-trimethylhydrazinium) propionate orotate**

Medizinische Verwendung von 3-(2,2,2-Trimethylhydrazinium)propionat-Orotat

Utilisation médicale d'orotate propionate 3-(2,2,2-trimethylhydrazinium)

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(30) Priority: **12.12.2007 EP 07122978**

(43) Date of publication of application:
**17.06.2009 Bulletin 2009/25**

(73) Proprietor: **Grindeks, a joint stock company
Riga 1057 (LV)**

(72) Inventors:
 • **Kalvins, Ivars
  Riga, 1006 (LV)**
 • **Vilskersts, Reinis
  Baldone (LV)**
 • **Liepins, Edgars
  Riga, 1007 (LV)**
 • **Dambrova, Maija
  Riga, 1002 (LV)**
 • **Birmans, Anatolijs
  Riga, 1006 (LV)**
 • **The other inventors have agreed to waive their
  entitlement to designation.**

(56) References cited:
**WO-A-2005/012233    GB-A- 2 105 992**

 • **DAMBROVA M ET AL: "Mildronate:
  Cardioprotective action through carnitine-
  lowering effect" TRENDS IN CARDIOVASCULAR
  MEDICINE, ELSEVIER SCIENCE, NEW YORK, NY,
  US, vol. 12, no. 6, 2002, pages 275-279,
  XP002409968 ISSN: 1050-1738**
 • **RUPP H ET AL: "THE USE OF PARTIAL FATTY
  ACID OXIDATION INHIBITORS FOR METABOLIC
  THERAPY OF ANGINA PECTORIS AND HEART
  FAILURE" HERZ, URBAN UND VOGEL,
  MUENCHEN, DE, vol. 27, no. 7, November 2002
  (2002-11), pages 621-636, XP001204997 ISSN:
  0340-9937**
 • **OL'BINSKAYA L I ET AL: "TREATING CARDIAC
  INSUFFICIENCY WITH MILDRONATE IN
  CORONARY HEART DISEASE PATIENTS"
  BIOSIS,, 1 January 1990 (1990-01-01),
  XP002478084**
 • **SESTI CASILDE ET AL: "Mildronate, a novel fatty
  acid oxidation inhibitor and antianginal agent,
  reduces myocardial infarct size without affecting
  hemodynamics" JOURNAL OF
  CARDIOVASCULAR PHARMACOLOGY, RAVEN
  PRESS, NEW YORK, NY, vol. 47, no. 3, 1 March
  2006 (2006-03-01), pages 493-499, XP008090058
  ISSN: 0160-2446**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present invention relates to 3-(2,2,2-trimethylhydrazinium) propionate orotate for use in the treatment of cardiovascular disease, selected from the group of myocardial infarction or arrhythmia.

Background Art.

Cardiovascular diseases (CVDs) are a group of disorders of the heart and blood vessels.

Myocardial infarction (heart attack) is a serious result of coronary artery disease. Myocardial infarction (MI) is the irreversible necrosis of heart muscle secondary to prolonged ischemia. A heart attack or myocardial infarction is a medical emergency in which the supply of blood to the heart is suddenly and severely reduced or cut off, causing the muscle to die from lack of oxygen. More than 1.1 million people experience a heart attack (myocardial infarction) each year, and for many of them, the heart attack is their first symptom of coronary artery disease. A heart attack may be severe enough to cause death or it may be silent. As many as one out of every five people have only mild symptoms or none at all, and the heart attack may only be discovered by routine electrocardiography done some time later.

A heart attack (myocardial infarction) is usually caused by a blood clot that blocks an artery of the heart. The artery has often already been narrowed by fatty deposits on its walls. These deposits can tear or break open, reducing the flow of blood and releasing substances that make the platelets of the blood sticky and more likely to form clots. Sometimes a clot forms inside the heart itself, then breaks away and gets stuck in an artery that feeds the heart. A spasm in one of these arteries causes the blood flow to stop.

Arrhythmias are related to the group of cardiovascular diseases. Arrhythmias are disorders of the regular rhythmic beating of the heart. Arrhythmias can occur in a healthy heart and be of minimal consequence. They also may indicate a serious problem and lead to heart disease, stroke or sudden cardiac death.

Arrhythmias can occur with a normal heart rate, or with heart rates that are slow (called bradyarrhythmias, less than 60 beats per minute), arrhythmias can also occur with rapid heart rates (called tachyarrhythmias, where heart rates faster than 100 beats per minute).

Arrhythmias may be caused by many different factors, including:

- Coronary artery disease
- Electrolyte imbalances in your blood (such as sodium or potassium)
- Changes in your heart muscle.
- Injury from a heart attack
- Healing process after heart surgery.

3- (2,2,2-Trimethylhydrazinium) propionate dihydrate is known as compound with cardioprotective properties (this substance being known under its International Nonproprietary Name of Meldonium). 3- (2,2,2-Trimethylhydrazinium) propionate dihydrate is disclosed in US 4481218 (INST ORGANICHESKOGO SINTEZA) 06.11.1984

It is well known that 3- (2, 2,2-trimethylhydrazinium) propionate as dihydrate is widely used for controlling carnitine and gamma-butyrobetaine concentration ratio and consequently the speed of fatty acid beta-oxidation in the body DAMBROVA M., LIEPINSH E., KALVINSH I. I. Mildronate: cardioprotective action through carnitine-lowering effect. Trends in Cardiovascular Medicine,. 2002, vol.12, no.6, p.275-279.

Due to these properties, Meldonium dihydrate is extensively applied in medicine as an anti-ischemic, stress-protective and cardioprotective drug in treating various cardio- vascular diseases and other pathologies involving tissue ischemia KARPOV R.S., KOSHELSKAYA O.A., VRUBLEVSKY A.V., SOKOLOV A.A., TEPLYAKOV A.T., SKARDA I., DZERVE V., KLINTSARE D., VITOLS A., KALNINS U., KALVINSH I., MATVEYA L., URBANE D.. Clinical Efficacy and Safety of Mildronate in Patients With Ischemic Heart Disease and Chronic Heart Failure. Kardiologiya. 2000, no.6, p.69-74.. In the treatment of cardiovascular diseases the mechanism of action of 3-(2,2,2-trimethylhydrazinium)propionate based on limitation of carnitine biosynthesis rate and related long-chain fatty acid transport limitation through mitochondria membranes SIMKHOVICH B.Z., SHUTENKO Z.V., MEIRENA D.V., KHAGI K.B., MEZHAPUKE R.J., MOLODCHINA T.N., KALVINS I.J., LUKEVICS E. 3-(2,2,2,-Trimethylhydrazinium)propionate (THP) - a novel gamma-butyrobetaine hydroxylase inhibitor with cardioprotective properties. Biochemical Pharmacology. 1988, vol.37, p.195-202., KIRIMOTO T., ASAKA N., NAKANO M., TAJIMA K., MIYAKE H., MATSUURA N.. Beneficial effects of MET-88, a γ-butyrobetaine hydroxylase inhibitor in rats with heart failure following myocardial infarction. European Journal of Pharmacology. 2000, vol.395, no.3, p.217-224.

GB 2 105 992 (MO MED INST PIROGOVA [SU]; INST ORGANICHESKOGO SINTEZA AK [SU], 7 April 1983) describes the use of meldonium for the treatment of arrhythmia (p. 1, l. 16-31, 44-63, 89-99; p. 3, tab. 3; p. 9, l. 12-29;

claim 1).

Orotic acid (OA), a naturally occurring substance, is a key intermediate in the biosynthetic pathway of pyrimidines.

An orotate is an insoluble organic salt formed by the binding of orotic acid with a mineral. Minerals such as calcium, potassium and magnesium are available in the orotate form. Mineral orotates are easily absorbed and utilised by the body. Minerals are actively transported from the digestive system into the blood stream by orotate salts. Once in the blood stream, the mineral separates from the orotate. The mineral is no longer bound to the orotate salt and is now free to exert its therapeutic action.

Orotic acid can improve the energy status of the recently infarcted myocardium. Orotic acid improves the tolerance of the recently infarcted heart to global ischemia. Magnesium orotate may reduce the severity of chronic myocardial dysfunction and structural damage in cardiomyopathy (cardiomyopathic hamsters). Magnesium orotate may improve exercise tolerance in patients with coronary artery disease and in trained athletes (humans). Magnesium orotate has only a weak inotropic effect, if any, on normal hearts. EP 0761224 A (WOERWAG PHARMA GMBH) 12.03.1997

Orotate salt of Meldonium is disclosed in EP 1667960 A (JOINT STOCK COMPANY GRINDEKS) 14.06.2006 as more stable substance comparatively with Meldonium dihydrate.

Disclosure of Invention

As it was known what Meldonium dihydrate has cardioprotective effect; however there are no data of cardioprotective effect of Meldonium salts.

To our surprise, by using Meldonium orotate in the treatment of cardiovascular diseases it shows unexpected effect, and was more effective as Meldonium dihydrate and Orotic acid in myocardial infarction models and Meldonium orotate in acute arrhythmia showed unexpected results in reduced fatal outcomes.

Best Mode for Carrying Out the Invention

The following examples further illustrate the invention.

Cardioprotective activity

Experiments were conducted on adult male Wistar rats with initial weight of 280-310g. During the experiment, the animals were kept in Standard crates in groups of 8. The feed was a standardized diet R70 (LABFOR, Lactamin AB, Sweden). The room temperature was kept at 21-23°C, relative humidity at $65 \pm 10\%$, 12 hour light/darkness cycle.

The experimental procedures were carried out in accordance with the guidelines of the European Community and local laws and policies and were approved by Latvian Animal Protection Ethical Committee, the Food and Veterinary Service.

Experiment 1

Isolated rat heart infarction model

Rats weighing approximately 300 g were randomly divided in 4 groups. The first group received saline by mouth (control group), the second group 120 mg/kg Meldonium dihydrate, the third orotic acid 100mg/kg and the fourth group 200 mg/kg Meldonium orotate by mouth for 14 days.

Rats were anesthetized with sodium pentobarbital (60 mg/kg) and heparin (1 IU/g) was administrated concomitantly. Hearts were excised and retrogradely perfused via aorta at a constant pressure of 50 mm Hg, with oxygenated Krebs-Henseleit buffer ( content in mmol/L: NaCl 118, Ca Cl$_2$ 2.52, MgCl$_2$ 1.64, NaHCO$_3$ 24.88, KH$_2$PO$_4$ 1.18, glucose 10.0, EDTA 0.05) pH 7.3-7.5 at 37°C.

A 5-0 polypropylene suture (Surgipro II, Syneture) was passed under the left anterior descending coronary artery and threaded through a small plastic tube to permit reversible occlusion of the coronart artery. Coronary flow was measured using an ultrasound flow detector (HSE) and PowerLab 8/30 system from ADInstruments.

Hearts were adapted for 20 minutes and occlusion was performed for 60 minutes by constricting threads through a plastic tube.

At the end of the 150 minute reperfusion, the left anterior descending coronary artery was relegated and the risk zone was delineated with 0.1 % methylene blue solution in Krebs-Henseleit buffer infused via the aorta root. Hearts were sectioned transversely from the apex to the base of 2 mm thickness and incubated in 1 % triphenyl-tetrazolium chloride in phosphate buffer (pH 7.4, 37°C) for 10 minutes to stain viable tissue red and necrotic tissue white. Afterward, the right ventricle was cut off and photos of the left-ventricle slices were made with a Minolta 7D photo camera. Computerized planemetric analysis of photographs was performed using Image-Pro Plus 4.5.1 software to determine the area at risk (AR) and area of necrosis (AN) expressed as percentage of the left ventricle (LV). Obtained values were then used to calculate the infarct size (IS) as percentage of risk area according to formula:

$$IS(\%) = \frac{AN}{AR} \times 100$$

Experimental groups were compared by one-way ANOVA (analysis of variance). P values less than 0.05 were considered to be significant.

Results of isolated rat infarction model by administration saline, Meldonium dihydrate, Orotic acid and Meldonium orotate for 14 days are summarizing in Table 1.

Table 1

Results of isolated rat infarction model

Table 1

|  | Infarct size, % of control |
|---|---|
| Control | 100.0±17.0 |
| Meldonium dihydrate | 76.9±5.9* |
| Orotic acid | 75.0±7.7* |
| Meldonium orotate | 49.1±6.1***,#,& |
| *p<0.05 relative to the control group; ***p<0.001 relative to the control group; # p<0.05 to Meldonium dihydrate group; & p<0.05 relative to orotic acid group. | |

For getting more information of properties of Meldonium orotate in the treatment of cardiovascular disease anti-arrhythmic experiment was made.

Anti-arrhythmic experiment

Experiment was conducted on adult male Wistar rats. During the experiment, the animals were kept in Standard crates in groups of 8. The feed was a standardized diet R3 (LABFOR, Lactamin AB, Sweden). The room temperature was kept at 21-23°C, relative humidity at 65±10%, 12 hour light/darkness cycle. The experimental procedures were carried out in accordance with the guidelines of the European Community and local laws and policies and were approved by Latvian Animal Protection Ethical Committee, the Food and Veterinary Service.

Experiment 2

An experimental acute arrhythmia was caused by introducing in rats solution of Calcium chloride.

Rats weighing 240-250 g randomized were divided into 3 groups, where in all groups there were 8 rats. During all 14 days rats got experimental preparations: Meldonium dihydrate or Meldonium orotate. The first group of 8 rats received drinking water (control group), the second group of 8 rats received Meldonium dihydrate (120 mg/kg p.o.) and the third group of 8 rats received Meldonium orotate (204 mg/kg p.o.). The last dose of experimental preparations animals got 24 h before the experiment. Before the experiment was started, male rats were anaesthetized with urethane 1g/ kg intraperitoneally. When narcosis started to influence to rat, there was cannulated left femoral vena for introducing of 10 % $CaCl_2$ and after that there was connected ECG's recoding device (ADInstrument PowerLab systems) and read ECG from the II lead. Processing of ECG was done using the programme Chart 5.5.

Experimental disturbances of the action of heart's rhythm were caused introducing solution of $CaCl_2$ in an arrhythmogenic dose (185 mg/kg), injecting solution of $CaCl_2$ with a regular rate (0.01ml/s). Registration of ECG was done from the start of introduction of $CaCl_2$'s solution and even 10 minutes after the end of introduction of $CaCl_2$'s solution.

The Table 2 shows the influence of Meldonium orotate on lethality caused from acute arrhythmias, induced by Calcium chloride.

| 3/8 | 2/8 | 0/8 |
|---|---|---|
| Control | Meldonium dihydrate | Meldonium orotate |

**Fatal outcome**

Table 2 Effect of Meldonium salts in acute arrhythmia.

Meldonium orotate showed unexpectedly good results, comparing with the control, showing that in these experimental groups there are no lethal cases.

**Claims**

1. Use of 3-(2,2,2-trimethylhydrazinium)propionate orotate for the manufacture of a medicament for the treatment of cardiovascular diseases, selected from the group of myocardial infarction or arrhythmia.

2. Use according to claim 1 where cardiovascular disease is myocardial infarction.

3. 3-(2,2,2-Trimethylhydrazinium)propionate orotate for use in the treatment of myocardial infarction.

4. Use according to claim 1 where cardiovascular disease is arrhythmia.

5. 3-(2,2,2-Trimethylhydrazinium)propionate orotate for use in the treatment of arrhythmia.

**Patentansprüche**

1. Anwendung von 3-(2,2,2-trimethylhydrazinium)propionatorotat für die Produktion von Arzneimittel für die Behandlung von kardiovaskulären Krankheiten, ausgewählt aus einer Gruppe, zu der Herzinfarkt (Myokardinfarkt) oder Herzrhythmusstörung gehören.

2. Anwendung nach Anspruch 1, soweit die kardiovaskuläre Krankheit der Herzinfarkt (Myokardinfarkt) ist.

3. 3-(2,2,2-trimethylhydrazinium)propionatorotat für die Behandlung von Herzinfarkt (Myokardinfarkt).

4. Anwendung nach Anspruch 1, soweit die kardiovaskuläre Krankheit die Herzrhythmusstörung ist.

5. 3-(2,2,2-trimethylhydrazinium)propionatorotat für die Behandlung von Herzrhythmusstörung.

**Revendications**

1. Utiliser du 3-(2,2,2-trimethylhydrazinium)propionate orotate pour la fabrication du médicament pour le traitement de maladies cardiovasculaires, choisies du groupe d'infarctus du myocarde ou d'arythmie.

2. Utiliser selon la réclamation 1 où la maladie cardiovasculaire est l'infarctus du myocarde.

3. 3-(2,2,2-Trimethylhydrazinium)propionate orotate pour le traitement de l'infarctus du myocarde.

4. Utiliser selon la réclamation 1 où la maladie cardiovasculaire est l'arythmie.

5. 3-(2,2,2-Trimethylhydrazinium)propionate orotate pour le traitement de l'arythmie.

**EP 2 070 529 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4481218 A
- GB 2105992 A
- EP 0761224 A
- EP 1667960 A

### Non-patent literature cited in the description

- **DAMBROVA M. ; LIEPINSH E. ; KALVINSH I. I.** Mildronate: cardioprotective action through carnitine-lowering effect. *Trends in Cardiovascular Medicine,* 2002, vol. 12 (6), 275-279
- **KARPOV R.S. ; KOSHELSKAYA O.A. ; VRUBLEVSKY A.V. ; SOKOLOV A.A. ; TEPLYAKOV A.T. ; SKARDA I. ; DZERVE V. ; KLINTSARE D. ; VITOLS A. ; KALNINS U.** Clinical Efficacy and Safety of Mildronate in Patients With Ischemic Heart Disease and Chronic Heart Failure. *Kardiologiya,* 2000, 69-74
- **SIMKHOVICH B.Z. ; SHUTENKO Z.V. ; MEIRENA D.V. ; KHAGI K.B. ; MEZHAPUKE R.J. ; MOLODCHINA T.N. ; KALVINS I.J. ; LUKEVICS E.** 3-(2,2,2,-Trimethylhydrazinium)propionate (THP) - a novel gamma-butyrobetaine hydroxylase inhibitor with cardioprotective properties. *Biochemical Pharmacology,* 1988, vol. 37, 195-202
- **KIRIMOTO T. ; ASAKA N. ; NAKANO M. ; TAJIMA K. ; MIYAKE H. ; MATSUURA N.** Beneficial effects of MET-88, a γ-butyrobetaine hydroxylase inhibitor in rats with heart failure following myocardial infarction. *European Journal of Pharmacology,* 2000, vol. 395 (3), 217-224